# EUROPÄISCHE PATENTSCHRIFT

(11) **EP 1 454 646 B1**
(45) Veröffentlichungstag und Bekanntmachung des Hinweises auf die Patenterteilung: **19.10.2005**
(21) Anmeldenummer: 03003999.4
(22) Anmeldetag: 24.02.2003
(51) Int. Cl.: A61M 5/30

(54) **Nadellose Spritze**
Needleless syringe
Seringue sans aiguille

(43) Veröffentlichungstag der Anmeldung: 08.09.2004
(73) Patentinhaber: Bünder Glas GmbH, 32257 Bünde (DE)
(72) Erfinder: Geiger, Andreas, 32278 Kirchlengern (DE)
(74) Vertreter: Schirmer, Siegfried

(56) Entgegenhaltungen:
- DE-U- 20 214 019
- US-A- 5 840 061
- US-A1- 2002 007 142
- US-A1- 2002 035 348

## Beschreibung

Die Erfindung betrifft eine nadellose Spritze, um ein Medikament unter hohem Druck unter die Haut eines Patienten zu injizieren.

Nadellose Spritzen haben zum einen den Vorteil, daß sie von empfindlichen Patienten leichter akzeptiert werden als mit Injektionsnadeln versehene Spritzen, und sind ferner dahingehend vorteilhaft, daß sich weder medizinisches Personal noch Patienten durch Kontakt mit einer Nadel einer gebrauchten Spritze infizieren können.

Aus der US 3527 212 ist eine nadellose Spritze mit einem äußeren Kunststoffkörper bekannt, in dessen zylindrischem Innenraum eine ein Medikament enthaltende, unter einem hohen Druck stehende Ampulle verschieblich gehalten ist. Die Ampulle weist eine mit einer zerstörbaren Membran verschlossene Öffnung auf, mit der die Ampulle gegen eine feststehend angeordnete Nadel verfahrbar ist, wobei die Nadel mit einer nach außen mündenden Abgabeöffnung der Spritze verbunden ist. Bei einer entsprechenden Verlagerung der Ampulle durchstößt die Nadel die Membran, und das Medikament wird unter hohem Druck durch die Abgabeöffnung nach außen gedrückt.

Bei diesem Stand der Technik ist nachteilig, daß die Ampulle aus medizinischen Gründen (Kontamination) aus Glas bestehen sollte, welches allerdings keinen hohen Innendruck aushält, so daß die Ampulle entweder sehr starkwandig oder aus einem anderen Material hergestellt sein muß. Außerdem ist ein Zusatzmedium zur Druckerzeugung erforderlich, z.B. CO₂, und es muß eine Membran vorgesehen sein.

Eine nadellose Spritze gemäß dem Oberbegriff des Anspruchs 1 wird im Dokument DE 202 14 019 offenbart.

Die Aufgabe der Erfindung besteht darin, eine nadellose Spritze bereitzustellen, bei der relativ dünnwandige Glasampullen zur Bevorratung des zu injizierenden Medikaments verwendbar sein sollen, der erforderliche hohe Druck aber dennoch in einfacher Weise aufzubauen sein soll.

Diese Aufgabe wird erfindungsgemäß durch eine nadellose Spritze mit dem Merkmalen des Anspruchs 1 gelöst.

Zweckmäßigerweise ist vorgesehen, daß der Kunststoffkörper um den zweiten Abschnitt herum dickwandiger als um den ersten Abschnitt ausgebildet ist.

Vorzugsweise ist der Kunststoffkörper außen und innen zylindrisch, wobei der erste Abschnitt einen größeren Innendurchmesser aufweist als der zweite Abschnitt.

Vorzugsweise ist vorgesehen, daß der Innendurchmesser der Glasampulle dem Innendurchmesser des zweiten Abschnitts entspricht. Hierbei kann weiter vorgesehen sein, daß die Länge des zweiten Abschnitts im wesentlichen mit der Länge der in der Glasampulle enthaltenen Flüssigkeitssäule zuzüglich der Länge des dem zweiten Abschnitt benachbarten Stopfens übereinstimmt.

In Ausgestaltung der Erfindung kann vorgesehen sein, daß der Kunststoffkörper, insbesondere im Bereich des ersten Abschnitts, mit Außengewinde versehen ist, auf das ein auf den Stempel wirkendes Betätigungsmittel aufschraubbar oder aufgeschraubt ist.

Die Erfindung wird nachfolgend anhand eines Ausführungsbeispiels unter Bezugnahme auf eine Zeichnung erläutert, in der:
Fig. 1 eine Querschnittsansicht einer erfindungsgemäßen nadellosen Spritze in einem Ausgangszustand zeigt, und
Fig. 2 die Spritze nach Fig. 1 in einem teilweise betätigten Zustand zeigt.

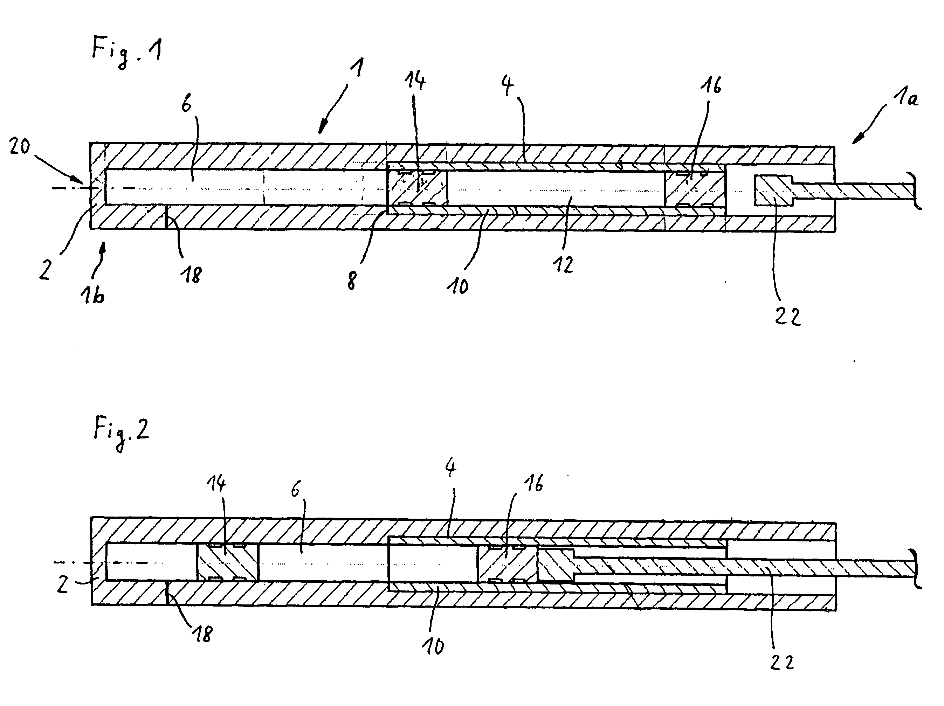

Fig. 1 zeigt eine Querschnittsansicht einer nadellosen Spritze mit einem außen und innen zylindrischen Kunststoffkörper 1 mit einem offenen Ende 1a und einem durch eine radiale Begrenzungswand 2 verschlossenen Ende 1b. Der Innenraum des Kunststoffkörpers 1 gliedert sich in einen ersten Abschnitt 4 und einen zweiten Abschnitt 6, wobei der Innendurchmesser des ersten Abschnitts größer ist als der des zweiten Abschnitts. Am Übergang zwischen den beiden Abschnitten 4 und 6 ist eine Schulter 8 ausgebildet.

Eine hohlzylindrische Glasampulle 10 ist in den ersten Abschnitt 4 unter Anlage gegen die Schulter 8 eingesetzt. Die Glasampulle enthält in ihrem Inneren ein Medikament 12 und ist an beiden Enden mit jeweils einem kolbenartigen Stopfen 14, 16 verschlossen.

Der Kunststoffkörper 1 weist mit Abstand zu dem ersten Abschnitt 4 eine seitliche Abgabeöffnung 18 auf, die zur Abgabe des Medikaments 12 dient, sowie im Bereich seiner Begrenzungswand 2 eine lediglich angedeutete Entlüftungsöffnung 20.

Ein Stempel 22 dient zum Eintreiben des dem zweiten Abschnitt 6 abgekehrten Stopfens 16, der sich in der Nähe des offenen Endes 1a des Kunststoffkörpers 1 befindet, wobei vorgesehen sein kann, daß der Stempel 22 mittels einer geeigneten Betätigungseinrichtung betätigt wird, die bspw. auf ein (nicht dargestelltes) Außengewinde des Kunststoffkörpers 1 aufschraubbar ist.

Die Wandstärke der Glasampulle 10 entspricht dem Radiusunterschied zwischen dem ersten und zweiten Abschnitt, d.h. der Innendurchmesser der Glasampulle ist im wesentlichen identisch mit dem Innendurchmesser des ersten Abschnitts 4 des Innenraums des Kunststoffkörpers 1, so daß eine Medikamentenabgabe in der nachfolgend beschriebenen Weise möglich ist.

Wie Fig. 2 zeigt, wird zunächst mit dem Stempel 22 der dem offenen Ende 1a benachbarte Stopfen 16 ohne bedeutenden Kraftaufwand in die Glasampulle eingeschoben, wobei aufgrund der Inkompressibilität des flüssigen Medikaments der zweite Stopfen 14 in den ersten Abschnitt 4 des Innenraums des Kunststoffkörpers 1 getrieben wird. Dieser Vorgang setzt sich fort, bis der Stopfen 14 gegen die Begrenzungswand 2 anliegt, wobei eingeschlossene Luft durch die Entlüftungsöffnung 20 entweichen kann. Wie man der Zeichnung entnimmt, ist die Abgabeöffnung 18 in einem solchen Abstand von der Begrenzungswand 2 angeordnet, daß sie dann, wenn der Stopfen 14 gegen die Begrenzungswand 2 anliegt, gerade nicht mehr durch den Stopfen verdeckt wird.

Wenn nach Erreichen der beschriebenen Zwischenposition der Stempel 22 rasch weiterbewegt wird, kann innerhalb des ersten Abschnitts 4 ein erheblicher Druck aufgebaut werden, so daß das Medikament 12 unter hohem Druck durch die Abgabeöffnung 18 abgegeben und injiziert wird. Dieser Vorgang setzt sich fort, bis der zweite Stopfen 16 gegen den ersten Stopfen 14 anliegt und die gesamte Menge des Medikaments 12 abgegeben worden ist.

Der Vorteil der erfindungsgemäßen Ausführung besteht darin, daß während der ersten Bewegungsphase die relativ druckempfindliche Glasampulle nur einem unwesentlichen Innendruck ausgesetzt wird, während der in der Injektionsphase erzeugte, hohe Innendruck ausschließlich innerhalb des von starken Außenwänden umgebenen zweiten Abschnitts 6 wirkt. Dies wird auch dadurch erreicht, daß die Länge des zweiten Abschnitts im wesentlichen mit der Länge der in der Glasampulle enthaltenen Flüssigkeitssäule zuzüglich der Länge des dem zweiten Abschnitt benachbartenn Stopfens entspricht.

Der Kunststoffkörper kann zur Verwendung einer weiteren Glasampulle wiederverwendbar sein, wofür er lediglich sterilisiert zu werden braucht.

Alternativ ist eine Einmalfunktion der nadellosen Spritze denkbar.

### Bezugszeichenliste:

- 1: Kunststoffkörper
- 1a: offenes Ende
- 1b: geschlossenes Ende
- 2: Begrenzungswand
- 4: erster Abschnitt
- 6: zweiter Abschnitt
- 8: Schulter
- 10: Glasampulle
- 12: Medikament
- 14: Stopfen
- 16: Stopfen
- 18: Abgabeöffnung
- 20: Entlüftungsöffnung
- 22: Stempel

## Patentansprüche

1. Nadellose Spritze mit einem Kunststoffkörper (1), der einen von einer Wandung umschlossenen Innenraum aufweist, welcher einen ersten, mit einem nach außen offenen Ende (1a) versehenen Abschnitt (4) und einen daran anschließenden zweiten Abschnitt (6) aufweist, mit einer in dem ersten Abschnitt (4) angeordneten, ein Medikament (12) enthaltenden Glasampulle (10), die an beiden Enden mit je einem kolbenartigen Stopfen (14, 16) verschlossen ist, wobei der zweite Abschnitt eine durch die Wandung des Kunststoffkörpers ausgebildete Abgabeöffnung (18) aufweist, und mit einem Stempel (22), mit dem der dem zweiten Abschnitt (6) abgekehrte Stopfen (16) in die Glasampulle (10) und in den zweiten Abschnitt (6) einschiebbar ist, **dadurch gekennzeichnet, dass** der zweite Abschnitt (6) eine Begrenzungswand (2) aufweist, die mit einer Entlüftungsöffnung (20) versehen ist, und dass der Abstand zwischen der Begrenzungswand (2) und der Abgabeöffnung (18) der Länge des dem zweiten Abschnitt (6) benachbarten Stopfens (14) entspricht.

2. Spritze nach Anspruch 1, **dadurch gekennzeichnet, dass** der Kunststoffkörper (1) um den zweiten Abschnitt (6) herum dickwandiger als um den ersten Abschnitt (4) ausgebildet ist.

3. Spritze nach Anspruch 1 oder 2, **dadurch gekennzeichnet, dass** der Kunststoffkörper (1) außen und innen zylindrisch ist, wobei der erste Abschnitt (4) einen größeren Innendurchmesser aufweist als der zweite Abschnitt (6).

4. Spritze nach einem der vorangehenden Ansprüche, **dadurch gekennzeichnet, dass** der Innendurchmesser der Glasampulle (10) dem Innendurchmesser des zweiten Abschnitts (6) entspricht.

5. Spritze nach Anspruch 4, **dadurch gekennzeichnet, dass** die Länge des zweiten Abschnitts (6) im wesentlichen der Länge der in der Glasampulle (10) enthaltenen Flüssigkeitssäule zuzüglich der Länge des dem zweiten Abschnitt (6) benachbarten Stopfens (14) entspricht.

6. Spritze nach einem der vorangehenden Ansprüche, **dadurch gekennzeichnet, dass** der Kunststoffkörper, insbesondere im Bereich des ersten Abschnitts (4), mit Außengewinde versehen ist, auf das ein auf den Stempel (22) wirkendes Betätigungsmittel aufschraubbar oder aufgeschraubt ist.

## Claims

1. Needleless syringe with a plastics material body (1) having an interior space, which is enclosed by a wall and which has a first section (4) provided with an outwardly opened end (1 a) and a second section (6) connected therewith, with a glass ampoule (10), which is arranged in the first section (4) and contains a medicament (12) and which is closed at both ends by a respective piston-like plug (14, 16), wherein the second section has a delivery opening (18) formed by the wall of the plastics material body, and with a plunger (22) by which the plug (16) facing away from the second section (6) can be pushed into the glass ampoule (10) and into the second section (6), **characterised in that** the second section (6) has a boundary wall (2) provided with a ventilation opening (20) and that the spacing between the boundary wall (2) and the delivery opening (18) corresponds with the length of the plug (14) adjacent to the second section (6).

2. Syringe according to claim 1, **characterised in that** the plastics material body (1) is constructed to be thicker walled around the second section (6) than around the first section (4).

3. Syringe according to claim 1 or 2, **characterised in that** the plastics material body (1) is externally and internally cylindrical, wherein the first section (4) has a greater internal diameter than the second section (6).

4. Syringe according to one of the preceding claims, **characterised in that** the inner diameter of the glass ampoule (10) corresponds with the inner diameter of the second section (6).

5. Syringe according to claim 4, **characterised in that** the length of the second section (6) substantially corresponds with the length of the liquid column contained in the glass ampoule (10) plus the length of the plug (14) adjacent to the second section (6).

6. Syringe according to one of the preceding claims, **characterised in that** the plastics material body is provided, particularly in the region of the first section (4), with an external thread onto which an actuating means acting on the plunger (2) can be screwed or is screwed.

## Revendications

1. Seringue sans aiguille avec un corps en plastique (1), qui présente un volume intérieur entouré par des parois, et comporte une première partie (4) pourvue d'une extrémité ouverte vers l'extérieur (1a) puis une seconde partie (6) qui s'y raccorde, avec une ampoule en verre (10) contenant un médicament (12) disposée dans la première partie (4), la deuxième partie de la seringue présente un orifice de sortie (18) pratiqué à travers la paroi du corps en plastique, et un poinçon (22), avec lequel le bouchon (16), situé à l'opposé de la deuxième partie (6), peut être enfoncé dans l'ampoule en verre (10) et dans la deuxième partie (6),
**caractérisée en ce que**
la deuxième partie (6) présente une paroi de limitation (2), pourvue d'un orifice de purge d'air (20), et la distance entre la paroi de limitation (2) et l'orifice de sortie (18) correspond à la longueur du bouchon (14) adjacent à la deuxième partie (6).

2. Seringue selon la revendication 1,
**caractérisée en ce que**
le corps en plastique (1) présente une paroi plus épaisse dans la deuxième partie (6) que dans celle de la première partie (4).

3. Seringue selon la revendication 1 ou 2,
**caractérisée en ce que**
le corps en plastique (1) est intérieurement et extérieurement cylindrique, la première partie (4) présentant un diamètre intérieur plus grand que celui de la deuxième partie (6).

4. Seringue selon l'une quelconque des revendications précédentes,
**caractérisée en ce que**
le diamètre intérieur de l'ampoule en verre (10) correspond au diamètre intérieur de la deuxième partie (6).

5. Seringue selon la revendication 4,
**caractérisée en ce que**
la longueur de la deuxième partie (6) correspond sensiblement à la longueur de la colonne de liquide contenue dans l'ampoule en verre (10) augmentée de la longueur du bouchon (14) adjacent à la deuxième partie (6).

6. Seringue selon l'une quelconque des revendications précédentes,
**caractérisée en ce que**
le corps en plastique, en particulier dans la région de la première partie (4), est pourvu d'un filetage extérieur sur lequel un moyen de commande agissant sur le poinçon (22) peut être vissé ou est vissé.
